# EUROPEAN PATENT APPLICATION

(11) **EP 4 234 388 A1**
(43) Date of publication of application: **30.08.2023**
(21) Application number: 23000035.8
(22) Date of filing: 27.02.2023
(51) Int. Cl.: B63G 8/00, B63B 59/08, B63G 8/38, B63G 8/39

(54) **A DEVICE FOR MONITORING THE TECHNICAL CONDITION OF UNDERWATER, STEEL PARTS OF PORTS' WHARF**

(30) Priority: 28.02.2022 PL 44050822
(71) Applicant: Uniwersytet Morski w Gdyni, 81-225 Gdynia (PL); Zarzad Morskiego Portu Gdansk SA, 80-955 Gdansk (PL)
(72) Inventor: Kaizer, Adam, 83-304 Smoldzino gm, Przodkowo (PL); Skrzypek, Michal, 81-363 Gdynia (PL); Wierzbicki, Pawel, 81-198 Kosakowo (PL); Mach, Blazej, 84-200 Wejherowo (PL); Troka, Agata, 83-032 Pszczolki (PL); Kulesza, Justyna, 84-207 Bojano (PL); Igansiak, Agnieszka, 81-461 Gdynia (PL)

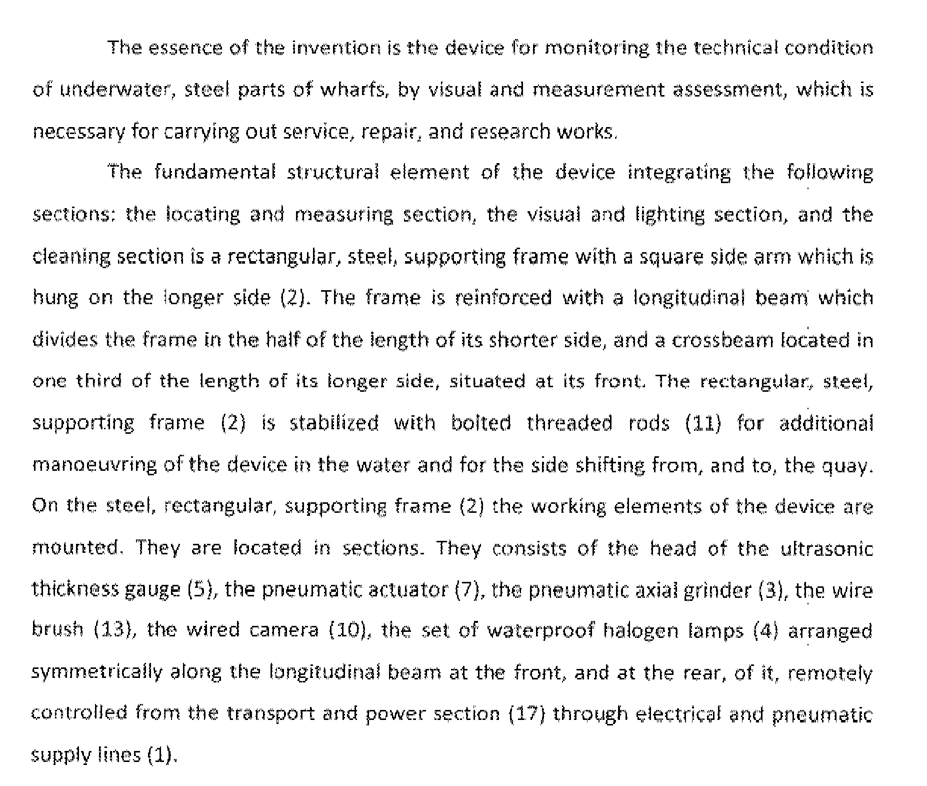

## Description

The essence of the invention is the device for monitoring the technical condition

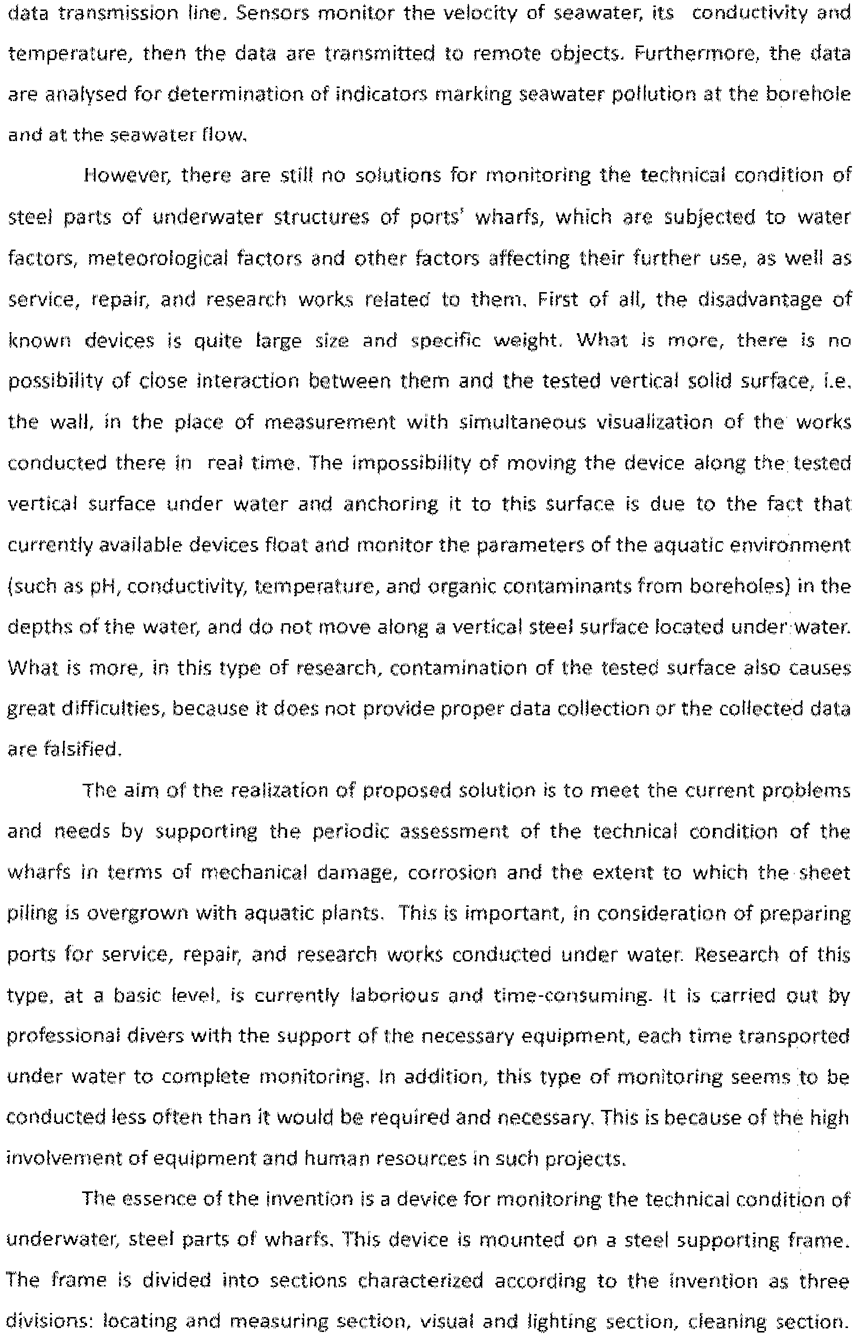

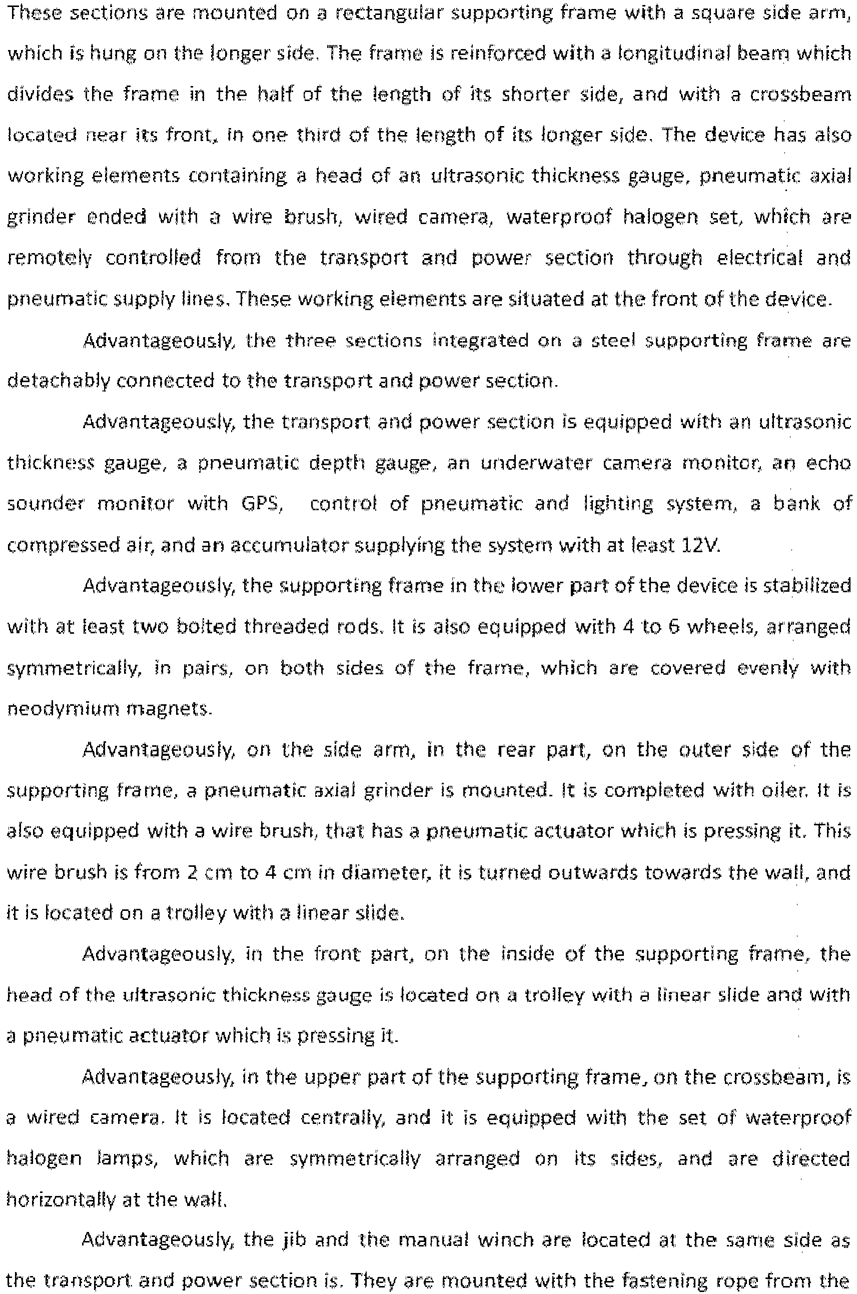

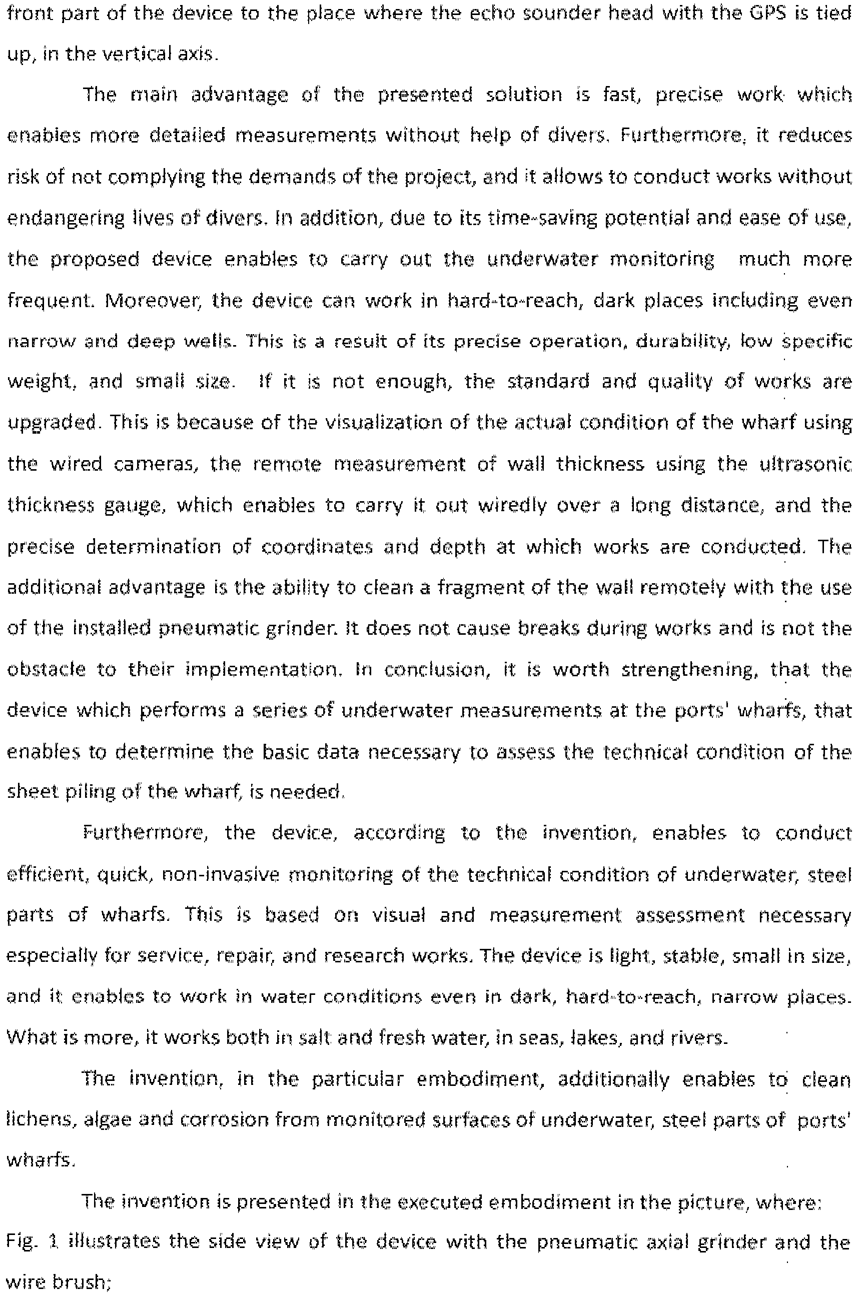

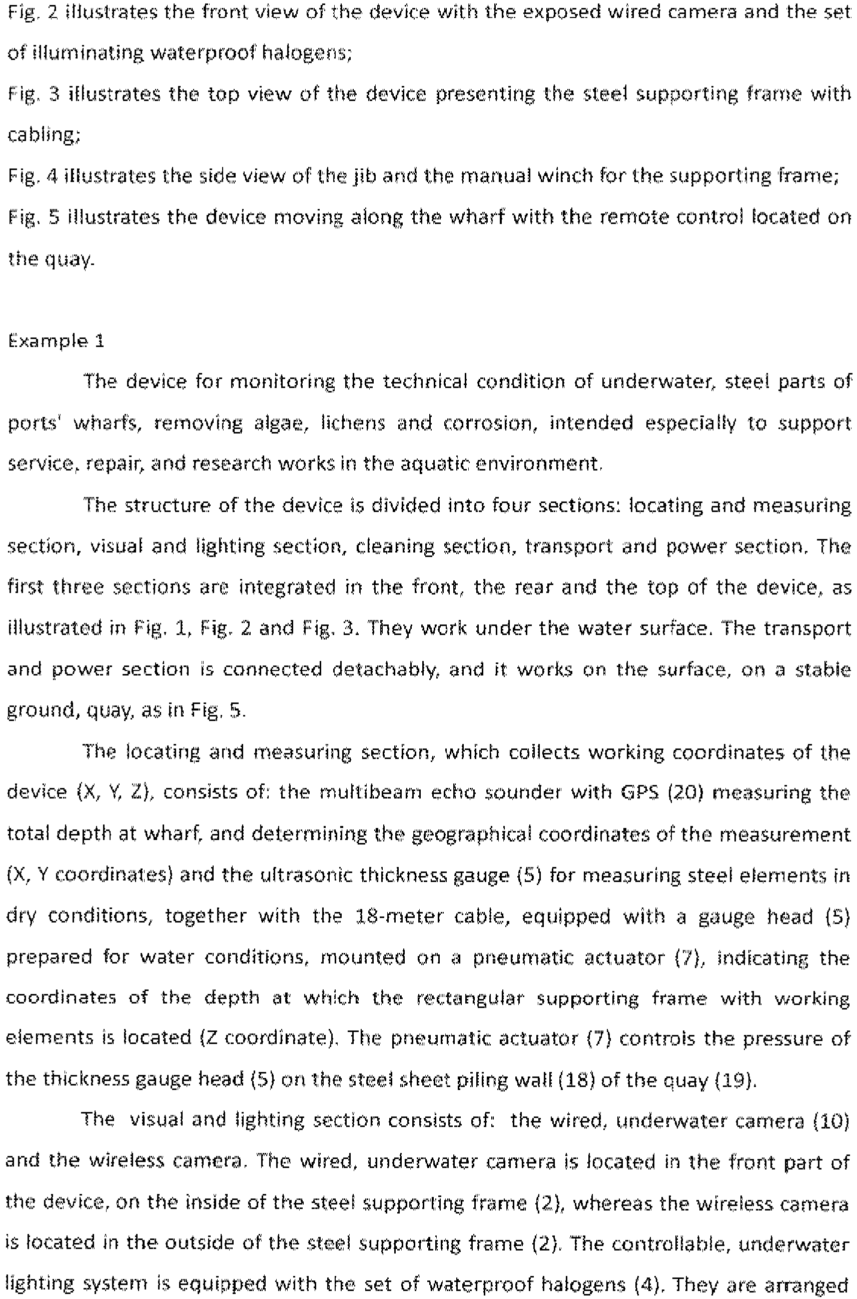

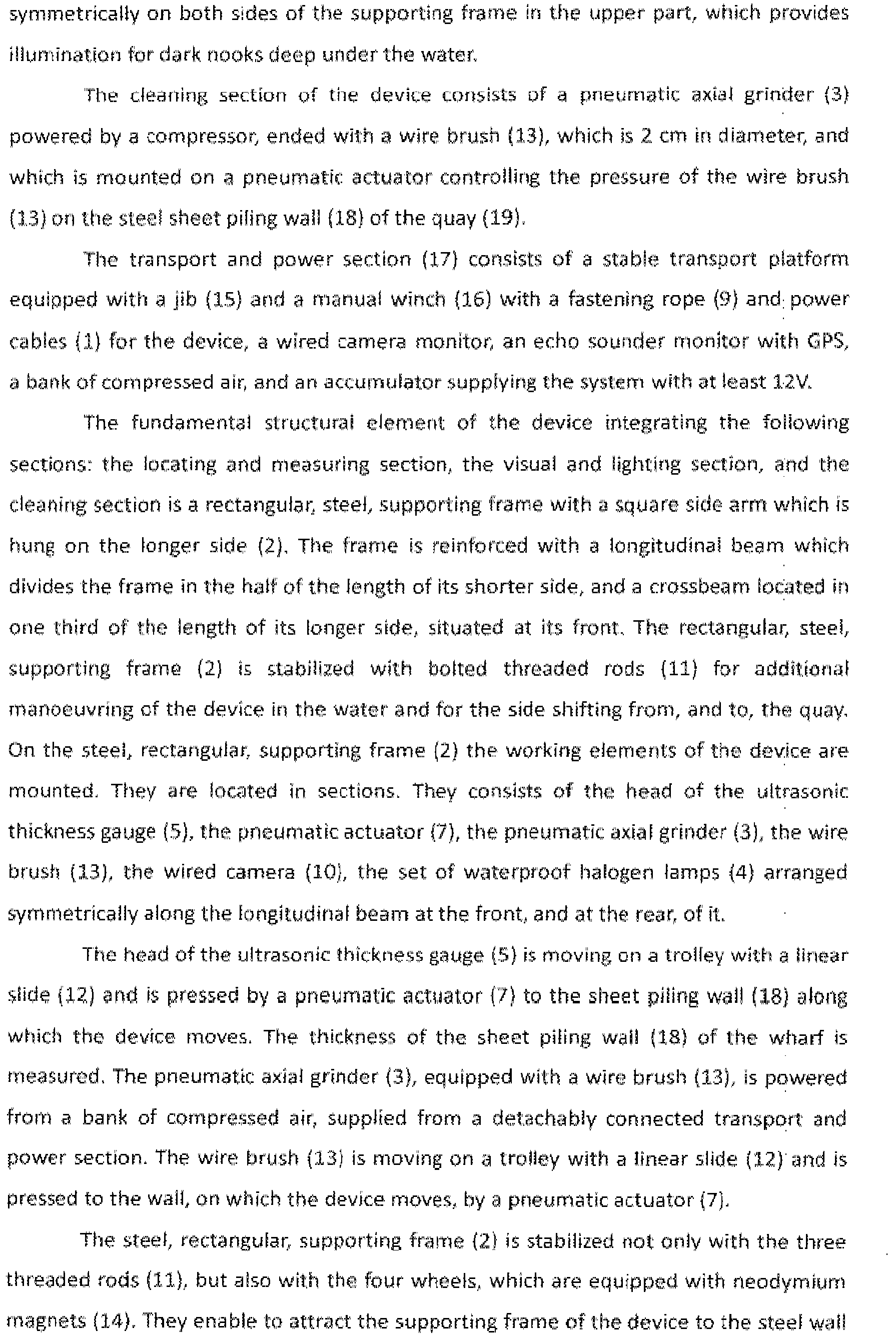

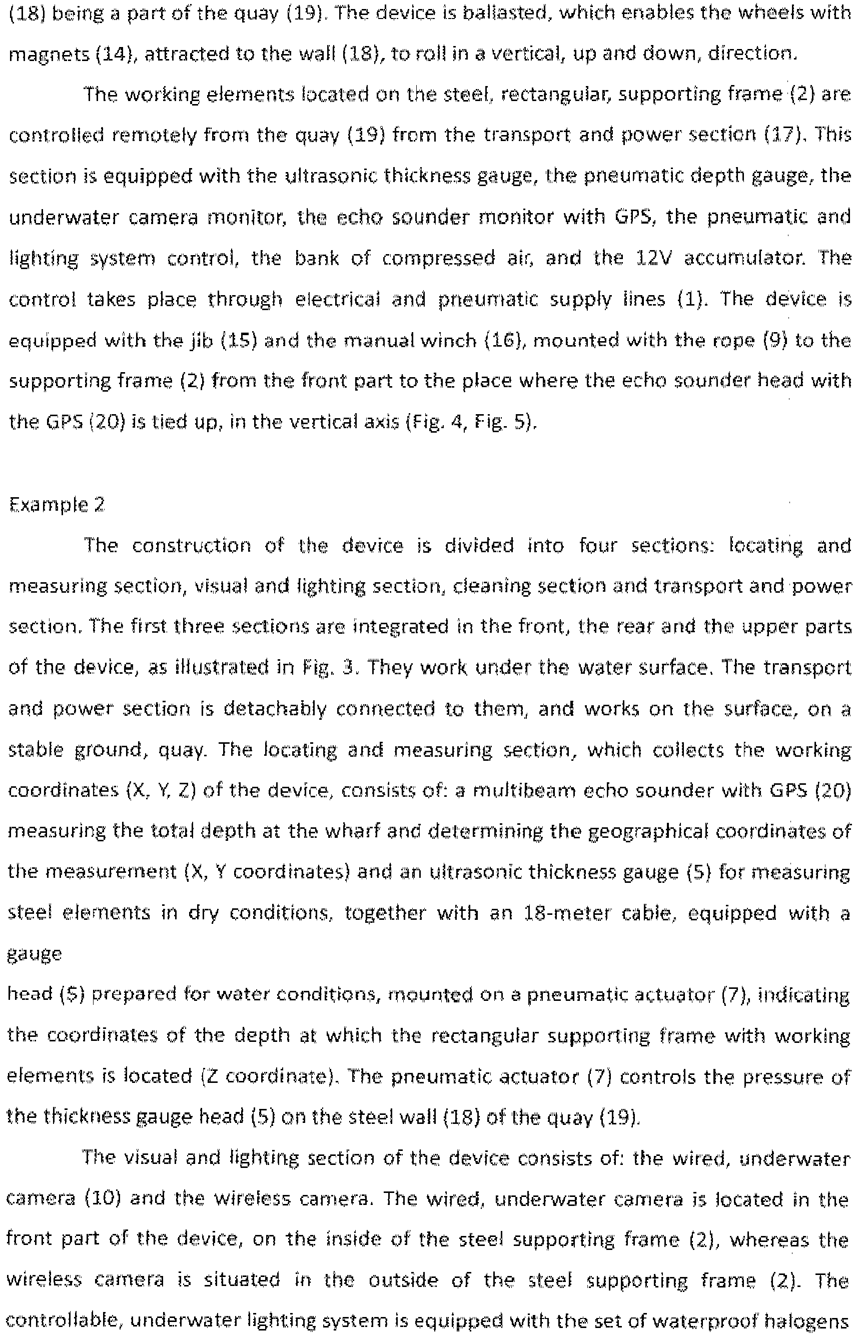

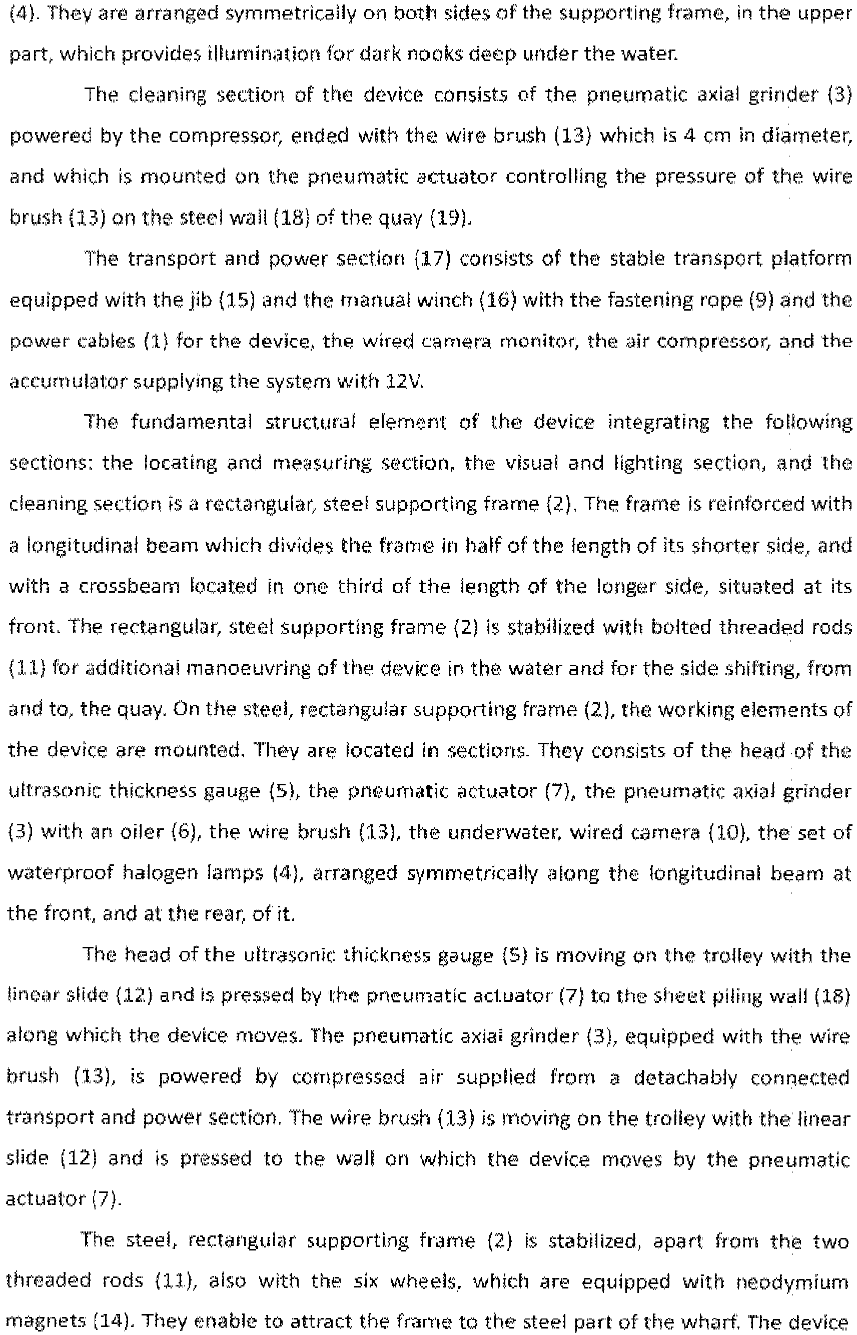

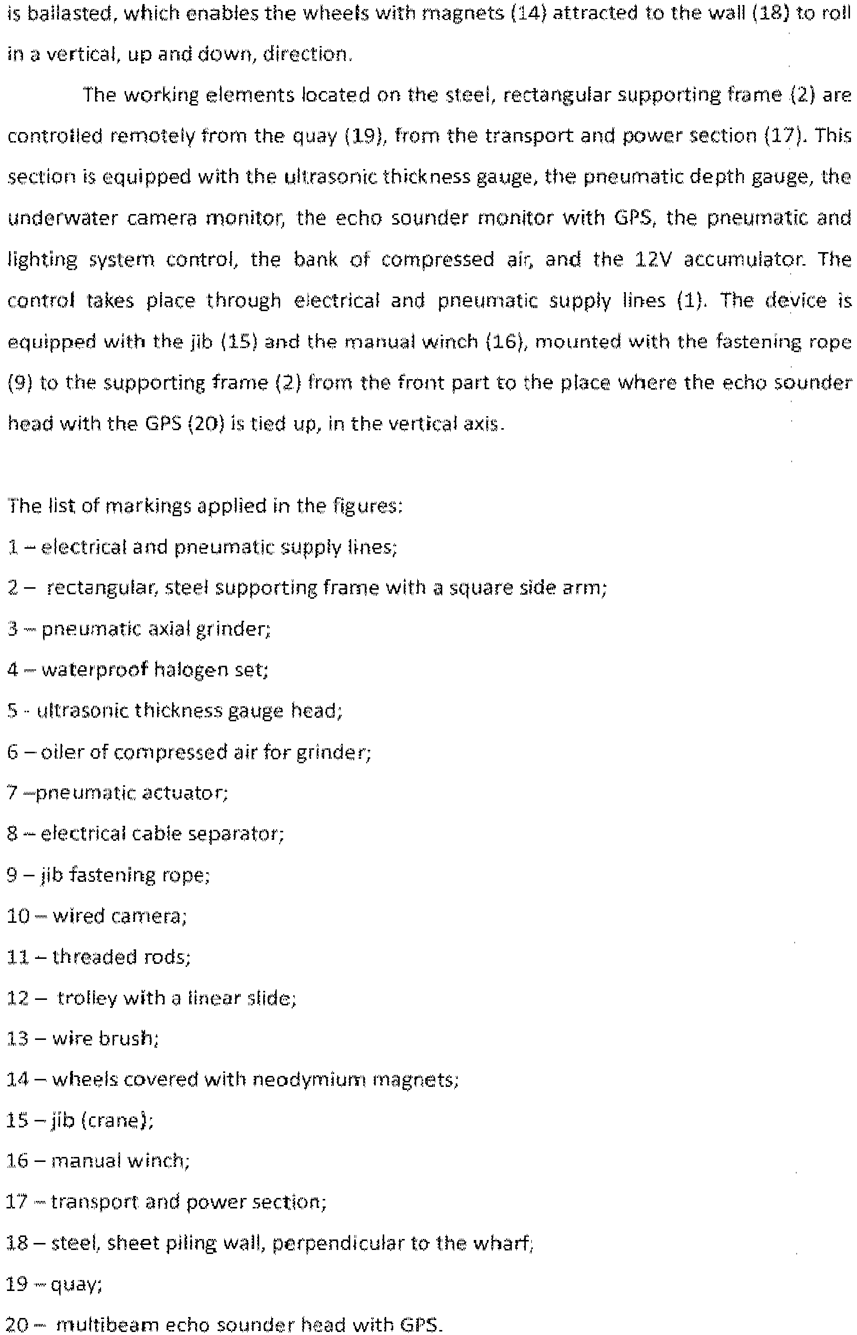

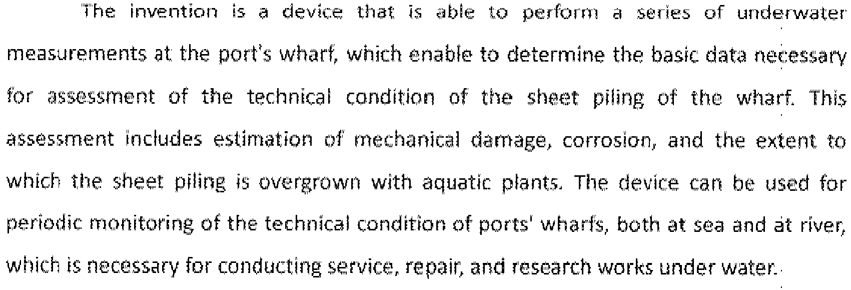

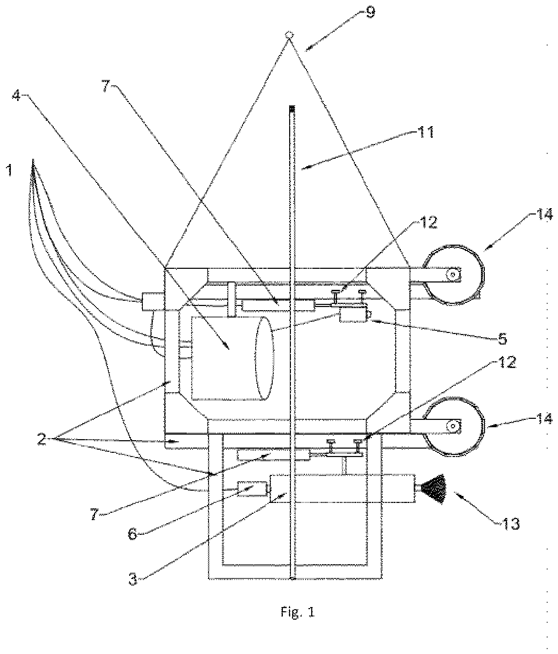

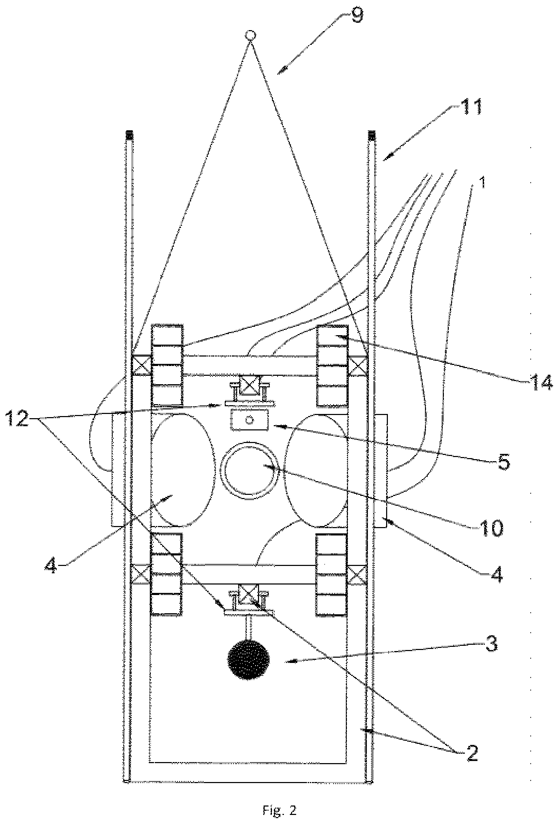

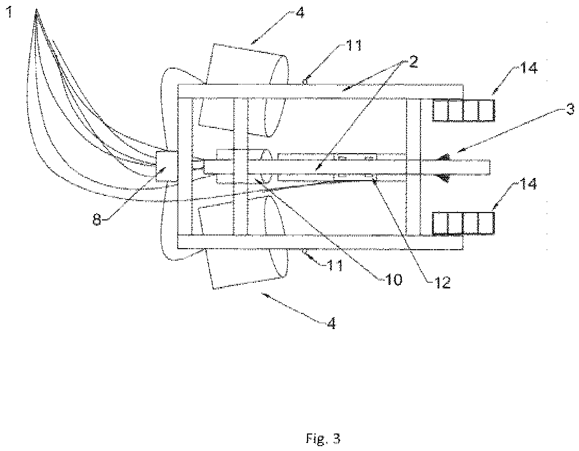

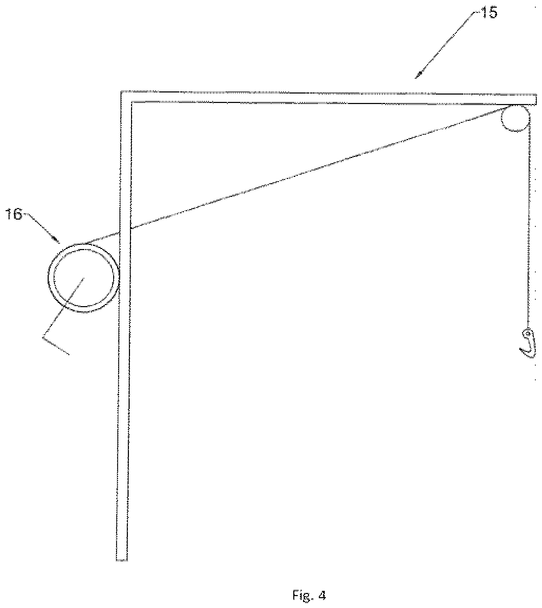

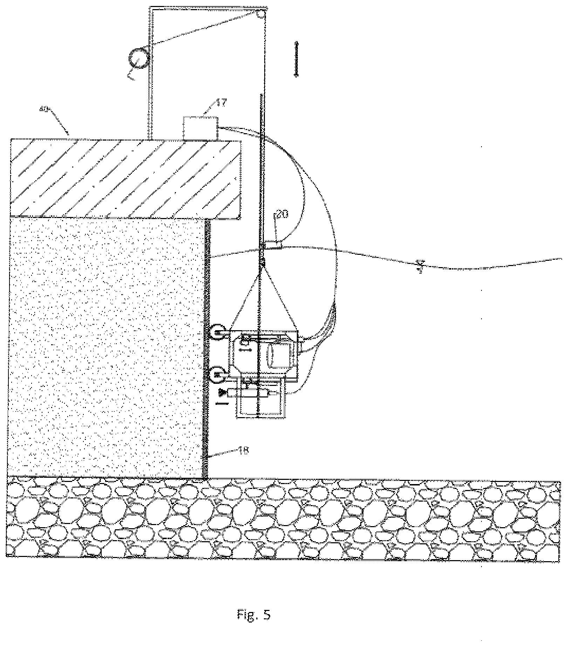

## Claims

1. The device for monitoring the technical condition of underwater, steel parts of parts'
